Europäisches Patentamt

European Patent Office

Office européen des brevets

(11) Publication number: 0 225 078
A2

# (12) EUROPEAN PATENT APPLICATION

(21) Application number: 86308748.2

(22) Date of filing: 11.11.86

(51) Int. Cl.⁴: **C12N 15/00** , C12N 1/14 , C12P 21/02 , C12N 9/34 , //(C12N1/14,C12R1:66)

(30) Priority: 20.11.85 US 799899

(43) Date of publication of application:
10.06.87 Bulletin 87/24

(84) Designated Contracting States:
AT BE CH DE ES FR GB GR IT LI LU NL SE

(71) Applicant: PANLABS, INC.
9725 Third Avenue, N.E.
Seattle, WA 98115(US)

(72) Inventor: Rambosek, John A.
8932 Ravenna Avenue N.E.
Seattle Washington 98115(US)
Inventor: Finkelstein, David A.
5535 56th Avenue, S.
Seattle Washington 98118(US)
Inventor: Leach, Jeanette
1401 5th Avenue, N. No. 703
Seattle Washington 98109(US)

(74) Representative: Harrison, David Christopher
et al
MEWBURN ELLIS & CO 2/3 Cursitor Street
London EC4A 1BQ(GB)

(54) Composite expression cassettes for fungal transformation.

(57) Novel hybrid DNA sequences useful for transforming filamentous fungi are provided. The hybrid DNA constructs include an expression cassette capable of controlling the expression of a structural gene encoding a selectable marker functional in fungi. The expression cassette can comprise a fungal promoter sequence and a fungal terminator sequence not natively associated with the selectable marker gene. After transformation into a fungal host, the hybrid DNA may be maintained as an extrachromosomal element or, alternatively, integrated into the fungal genome.

A plasmid, pGAN, embodying a preferred construct of the present invention was deposited at the American Type Culture Collection on 19 November 1985, and designated Accession No.53331.

## COMPOSITE EXPRESSION CASSETTES FOR FUNGAL TRANSFORMATION

The advent of recombinant DNA technology has brought with it an ever increasing need for improved methods of transferring DNA between organisms. Perhaps the cornerstone of DNA exchange mechanisms is transformation, conceptually the simplest form of genetic transfer. For some time, it has been recognized that a successful transformation protocol requires stabilization of DNA through integration or autonomous replication, and a means for ensuring that the genetic transfer has been successful, typically through selection.

Traditional methods for the development of autonomous vectors have generally included one or more of the following techniques:

1) utilization of a large portion of a naturally occurring host plasmid as the basic vector framework (e.g., 2 micron yeast vectors for yeast or mitochondrial plasmids for certain fungi);

2) incorporating into the vector autonomously replicating sequences (ARS), the identification of which can be accomplished by the shotgun cloning of genomic fragments into the organism of choice and then searching for extraneous DNA sequences passed on to progeny; or

3) utilization of replicons first identified by their ability to function as such in a heterologous system.

Importantly, while some of these methods have been useful in the identification of ARS sequences for yeast, similar approaches in other eukaryotes, such as filamentous fungi, have been only moderately satisfactory. This has in-turn inhibited the development of improved transformation protocols for such organisms.

One of the most frequently utilized transformation procedures relies on complemention, the ability of an organism with an auxotrophic mutation to survive in the presence of an appropriate DNA sequence from a prototrophic organism.

Although quite useful for experimental purposes, complementation is often inadequate for transforming industrial organisms. Frequently, industrial organisms do not contain known auxotrophic markers. Of course, such markers may be introduced by mutagenesis, but this process is not yet easily controllable and can often cause genetic damage throughout the organism's entire genome.

Thus, there exists a need for vectors capable of transforming lower eukaryotes, such as filamentous fungi. These vectors should be stable, have a broad host range, and be capable of accepting other DNA sequences for the ultimate introduction of heterologous structural genes into hosts. The present invention fulfills these needs.

Brief Description of Relevant References

Collins, et al., Cell (1981) 24:443-452 describes the characterization of a novel plasmid found in mitochondria from N. crassa. Case et al., Proc. Natl. Acad. of Sci. U.S.A. (1979) 76:5259-5263 and Stahl et al., Proc. Natl. Acad. of Sci. U.S.A. (1982) 79:3641-3645 describe the transformation, utilizing hybrid plasmid DNA, of N. crassa and Podospora , respectively. Kelly and Hynes, EMBO J. (1985) 4:475-479, report transformation of A. niger using the amdS gene of A. nidulans. Paietta and Marzluf, Mol. and Cell. Biol. (1985) 5:1554-1559 report that bacterial sequences may not be required to transform N. crassa. Nunberg et al., Mol. and Cell. Biol. (1984) 4:2306-2315, which is incorporated herein by reference, demonstrate the cloning of the A. awamori glucoamylase gene, and Boel et al., EMBO J. (1984) 3:1581-1585 describe two intervening sequences in the A. niger glucoamylase gene. The nucleotide sequence of a 2.7 kb genomic fragment containing the N. crassa am (NADP-specific glutamate dehydrogenase) gene is reported by Kinnaird and Fincham, Gene (1983) 26:253-260 and an unstable mutant gene at the am locus is reported by Rambosek and Kinsey, Gene (1984) 27:101-107. Hughes et al., Proc. Natl. Acad. Sci. U.S.A. - (1983) 80:1053-1057 describe difficulties associated with producing a functional neomycin phosphotransferase gene (obtained from Tn903) in N. crassa.

## SUMMARY OF THE INVENTION

Novel stable hybrid DNA constructs are provided that are useful for transforming filamentous fungi. These vectors can exist stably as extrachromosomal elements or, alternatively, can become integrated into the host genome. More specifically, the novel vectors include an expression cassette controlling the expression of a selectable marker. The expression cassette contains a fungal promoter, and in some embodiments a fungal terminator sequence and a prokaryotic origin of replication as well. The promoter and/or terminator sequences can be obtained from a fungal species different from the host species, and such sequences are generally not natively associated with the selectable marker. Expression cassettes controlling expression of additional structural genes can also be included. The resulting constructs can transform filamentous fungi of various species for numerous utilities, including overproduction of proteins of interest, such as glucoamylase.

## DESCRIPTION OF THE SPECIFIC EMBODIMENTS

In accordance with the present invention, novel hybrid DNA constructs are provided for use in stably transforming filamentous fungi, either by maintenance as an extrachromosomal element in the fungal host or by incorporation into the fungal genome. The hybrid DNA composition includes a plasmid an expression cassette which controls the expression of a structural gene encoding a polypeptide capable of acting as a selectable marker in fungi. The expression cassette comprises a fungal promoter sequence, and in some instances, a fungal terminator sequence. Commonly, neither sequence is natively associated with the selectable marker gene. The plasmid may also include prokaryotic DNA segments, such as a bacterial origin of replication.

By utilizing hybrid DNA compositions of the present invention, one can readily transform various species of filamentous fungi. This is typically accomplished by forming a vector by joining a DNA segment of interest with an expression cassette of the present invention. The vector is then utilized to transform a fungus, and the resulting transformants are grown under conditions requiring, for survival, functional expression of a selectable marker gene in the expression cassette. Typically, only transformants containing the hybrid DNA composition, either as an extrachromosomal element or incorporated into the host genome, will survive. When a second expression cassette with a structural gene is present, either in composite or native form, polypeptides encoded by the structural gene may be produced in desired quantities.

By way of example and not of limitation, one embodiment of the hybrid DNA compositions of the present invention included the following DNA sequences: an E. coli origin of replication and an expression cassette containing the 5' and 3' regions that natively flank the structural gene encoding N. crassa am enzyme, which regions in this embodiment control the expression of the neomycin phosphotransferase II - (NPT II) gene from the bacterial transposon Tn5. By transformation, this hybrid DNA composition in the form of a shuttle vector was able to stably transform A. niger and P. chrysogenum for the trait of G418 resistance. Moreover, two classes of transformants were obtained. One class was integrated into the host genome, while the second was found to be autonomously replicating. The autonomous replication was shown by Southern hybridization and also by the fact that the vector was recoverable in E. coli, in many instances unmodified. Thus, although the shuttle vector contained no sequences originating in either A. niger or P. chrysogenum, it was capable of stable transformation in those fungi. Similarly, when the expression cassette included the promoter and terminator sequences of the A. niger glucoamylase gene controlling the Tn5 NPTII gene, all placed in a commonly utilized plasmid, pUC8 the resulting vector was capable of transforming P. chrysogenum, as well as A. niger.

The source of promoter and terminator DNA sequences and transformation hosts are the filamentous fungi, particularly the class Ascomycetes. This class includes such representative genera as Neurospora, Aspergillus and Penicillium. Particular species include, for example, A. niger, N. crassa, and P. chrysogenum.

The expression cassettes can include promoter and terminator sequences from a naturally-occurring fungal gene. Of particular importance are promoters recognized by the host fungus to permit efficient transcription. In many cases, the promoters may be controlled by various regulatory signals. Typically, the preferred promoters will be those involved in metabolism or catabolism in a filamentous fungal host. Other promoters include those that are regulated by the growth stage of the host, such as vegetative growth, conidia formation, etc., or also protective against heat shock. By way of example and not of limitation, suitable promoter and terminator regions include those associated with the A . niger β-tubulin A. niger glucoamylase, A. niger phosphoglycerate kinase A. awamori glucoamylase, and N . crassa glutamate dehydrogenase structural genes.

The expression cassette is typically obtained from filamentous fungi by first cloning a DNA segment containing a fungal gene, preferably one transcribed in large quantities and, if desired, one that encodes a secreted protein. This DNA segment may be obtained in a variety of means, most commonly by first determining the N-terminal amino acid sequence of the gene's protein product and then making a series of

3

DNA segments based on the predicted nucleotide sequence for utilization as probes of cDNA or genomic libraries to identify and clone DNA fragments containing the desired gene and flanking regions. (See, for example, Nunberg et al., Mol. Cell. Biol. (1984) 4:2306-2315 and Kinnaird et al., Gene (1982) 20:387-396, both of which are incorporated herein by reference.)

The DNA fragment containing the promoter, selectable marker structural gene and terminator regions will generally be under about 5kb, usually under 3kb, but may be smaller depending primarily on the size of the structural gene. Generally, the promoter region will comprise about 50-500bp, more usually about 300bp. The terminator regions may be somewhat smaller, but also vary in size depending upon the particular instance.

Once the DNA fragment containing the promoter and/or terminator regions has been isolated, it is usually sequenced to determine the general location of the desired sequences. Through procedures well-known to those skilled in the art, these sequences may then be cut and ligated into various constructions, such as to form the expression cassettes of the present invention. By providing cohesive ends, butt ends or cleavage sites, it is possible to easily join a DNA sequence encoding a structural gene in proper orientation with the promoter and terminator sequences. Thus, one can control the expression of a desired gene, such as the selectable marker gene. Preferably, the DNA sequences between the promoter and terminator regions may provide for restriction by different enzymes to simplify the joining of the various DNA sequences. Although it is preferable to utilize the promoter and terminator regions that natively flank the same gene, regions from two different genes may be used if desired .

The selectable markers may be any of a large number of known markers, so long as they may be selected for when they are functional in the fungi. When placed in proper orientation in the expression cassette, the markers will provide for selection pressure, ensuring that the host retains the construct and does not become cured. Suitable markers may include biocidal resistance, e.g., antibiotic resistance, heavy metal resistance, etc., complementation of auxotrophy, or the like. Alternatively, a marker may be introduced to provide for viral immunity. In some instances, it may be desirable to have more than one marker present, depending upon the need for varying selective pressures.

The DNA segment containing a prokaryotic origin of replication may be obtained from any of a number of well-known sources. Bacterial plasmids are preferred, because of their ease of isolation and manipulation. By way of example, and not of limitation, suitable origin of replication sequences can be obtained from pBR322, pUC8, pUC18, all of which are well-known and available in the art.

When the expression cassette with a selectable marker is joined to a prokaryotic origin of replication DNA sequence, the resultant vector can act as a shuttle vector, and is highly suitable for manipulation to incorporate additional DNA sequences. These additional DNA sequences can include a variety of regulatory signals, including in addition to the promoter and terminator regions, ribosomal start sites, sequences coding for leader peptides recognized in filamentous fungi, sequences capable of amplifying various genes of interest by reiteration in filamentous fungi, etc. Those skilled in the art will realize that the vectors may be constructed in any order, based on the ability to clone and select for the fragments, the availability of various restriction sites, and the availability of combinations of regulatory signals and genes.

Once completed, the vector can be used to transform filamentous fungi, which are grown under selective conditions allowing solely for expansion of transformed hosts. The resulting transformants may then be grown one or more generations and yet retain the DNA construct either extrachromosomally or incorporated into the genome. Thus, the fungal host may be grown through a plurality of generations in different fungal stages, while maintaining the hybrid DNA intact. If desired, the hybrid DNA constructs can be reisolated from the host fungus and used for further manipulations or transformation. In some instances, it may be desirable to reduce the size of the vector to allow incorporation of additional expression cassettes with structural genes or intact gene for expression in the host.

These additional structural genes introduced may provide for a variety of products ranging from single amino acids to high molecular weight polypeptides. As desired, the genes may provide for production of enzymes capable of modifying non-proteinaceous products, such as antibiotics. Additional polypeptide products of interest include, by way of example, commercial prokaryotic and eukaryotic enzymes, mammalian hormones, toxins, vaccines and the like. Certain genes encoding naturally occurring proteins, such as the enzyme glucoamylase, or mutants thereof, may be introduced by transformation with the vectors of the present invention. This provides an additional means to control the production of such proteins (e.g., by increasing the copy number of a natural gene or when the expression cassette includes a different promoter than naturally present with the gene encoding the protein, in either case permitting 3-5 times, or greater, over production if desired).

The following examples are offered by way of illustration and not by way of limitation.

## EXPERIMENTAL

For the following experiments, the DNA manipulations were carried out in accordance with well-known procedures, such as described in Maniatis, T. et al., "Molecular Cloning, A Laboratory Manual," Cold Spring Harbor Laboratory, U.S.A. (1982), which is incorporated herein by reference.

All enzymes and plasmids, unless otherwise stated, are available from various commercial sources, such as New England Biolabs, Beverly, Massachusetts; Collaborative Research, Waltham, Massachusetts; Miles Laboratories, Elkhart, Indiana; Boehringer Biochemicals, Inc., Indianapolis, Indiana and Bethesda Research Laboratories, Rockville, Maryland. Buffers and reaction conditions for restriction enzyme digestion were used according to recommendations supplied by the manufacturer for each material, unless otherwise indicated. All cited publications and patents are specifically incorporated herein by reference.

The following stock solutions were utilized:

1. KMP = 0.7 M KCl/ 0.8 M mannitol/ 20 mM $KPO_4$, pH 6.3.
2. KMPC = KMP plus 50 mM $CaCl_2$ (Ca added just before use).
3. Heparin = KMPC with 5 mg heparin/ml, made fresh before each use.
4. PPC = 40% Polyethylene glycol (Sigma 3500)/20 mM $KPO_4$, (pH 6.3,)/50 mM $CaCl_2$.
5. Regeneration Agar

| | |
|---|---|
| 20 × Mineral salts | 50 ml |
| Tryptone | 5 g |
| Yeast extract | 5 g |
| Glucose | 10 g |
| Mannitol | 236.86 g. |

Adjusted to one liter with water, heated when necessary to dissolve the mannitol. To each 125 ml media bottle, 1.5 grams agar and 50 ml of above solution were added.

6. Overlay agar. 1% peptone and 0.7% agar, made in 50 ml aliquots.
7. 20 × mineral salts.

| | |
|---|---|
| $Na_2NO_3$ (sodium nitrate) | 120 g |
| KCl | 10.4 g |
| $MgSO_4.7H_2O$ | 10.4 g |
| $KH_2PO_4$ | 30.4 g |
| Vogel's trace elements | 2.0 ml |
| Deionized water | to one liter. |

Each salt should be dissolved completely before adding the next. Stored at room temperature with 2 ml of chloroform as preservative. Used at 50 ml per liter.

8. Vogel's trace elements:

95 ml deionized water
5 g citric acid (NOT sodium salt)
5 g $ZnSO_4.7H_2O$
1 g $Fe(NH_4)_2 (SO_4)_2. 6H_2O$
0.25 g $CuSO_4$
0.05 g $MnSO_4.H_2O$
0.05 g $H_3BO_3$
0.05 g $Na_2MoO_4.2H_2O$.

Each ingredient is dissolved before adding the next, stored at about 4°C, and used at 0.1 ml per liter of medium.

9. Complete Medium

| | |
|---|---|
| 20 × Mineral Salts | 50 ml |
| Tryptone | 5 g |
| Yeast Extract | 5 g |
| Glucose | 10 g |
| Water | qv 1 L |

## A. ISOLATION OF THE A. NIGER GLUCOAMYLASE GENE

A Sau3A-partial library of A. niger DNA (from strain ATCC 1015) was constructed in lambda phage EMBL4 (A. M. Frischauf, H. Lehrach, A. Poustra and N. Murray "Lambda Replacement Vectors Carrying Polylinker Sequences". J. Mol. Biol. 170:827-842 (1983), available from Promega). The glucoamylase gene was isolated from this library by screening plaques with a 26mer probe - (GCGGACGGTGCTTGGGTGTCGGGCGC). The sequence of the probe was taken from the published glucoamylase sequence: J.H. Nunberg, J.R. Meade, C. Cole, F.C. Lawyer, P. McCabe, V. Schweichart, R. Tal, V.P. Wittman, J.E. Flatgaard, and M.A. Innis "Molecular Cloning and Characterization of the Glucoamylase Gene of Aspergillus awamori", Molecular and Cellular Biology 4:2306-2315 (1984). The phage used in these experiments was termed lambda AGIII. This phage contains the complete glucoamylase gene as part of a 19kb insert. The restriction map agrees with the published information about the gene from A. niger strain BU-1 and A . awamori (NRRL 3112): E. Boel, M.T. Hansen, I. Hoegh and N.P. Fiil, "Two different types of intervening sequences in the glucoamylase gene from Aspergillus niger ", The EMBO Journal 3:1581-1585 (1984); and J.H. Nunberg, J.R. Meade, G. Cole, F.C. Lawyer, P. McCabe, V. Schweichart, R. Tal, V.P. Wittman, J.E. Flatgaard, and M.A. Innis "Molecular Cloning and Characterization of the Glucoamylase Gene of Aspergillus awamori", Molecular and Cellular Biology 4:2306-2315 (1984). (All of the above references are specifically incorporated herein by reference.)

## B. CONSTRUCTION OF VECTORS

1. pPLI: The 2kb EcoRI-BamHI fragment of pJR2 (containing the 5' region and coding sequences plus a portion of 3' non-coding sequences of the N . crassa am gene; nucleotides 1-2034, numbered according to J.H. Kinnaird and J.R.S. Fincham "The complete nucleotide sequence of the Neurospora crassa am (NADP-specific glutamate dehydrogenase) gene," Gene 26:253-260 (1983) and obtained from J. Kinsey, University of Kansas Medical Center, Kansas City), was isolated and cloned between the EcoRI and BamHI sites of pBR322.

2. pPLI-R: pPLI was cut with Cla I (at nucleotide 283, numbered according to Kinnaird and Fincham, supra) and the cohesive ends were made flush by incubation with reverse transcriptase and a mixture of the 4 deoxyribonucleotide triphosphates. Then, the plasmid was incubated with synthetic phosphorylated EcoRI linkers (GGAATTCC), the material cut with EcoRI, and the plasmid ligated.

3. pUTX27: pBR322 was cut with EcoRI, filled with the Klenow fragment of DNA polymerase, and reclosed. This removed the EcoRI site.

4. pEVI: pUTX27 was cut with BamHI and phosphatased. This was ligated to the ∿300 bp BamHI-EcoRI fragment of pPLI-R (which contains the promoter and transcription start site of the am gene) and the ∿600 bp EcoRI-BamHI fragment from pJR2 (nucleotides 2030-2644 numbered according to Kinnaird and Fincham, supra) which contains the sequences surrounding the polyA addition site of the am gene. The resulting plasmid contains a unique EcoRI site between the transcription start and stop sites of the am gene.

5. pRRneo: The BglII-AvaI fragment of pNEO (nucleotides 1516-2520 numbered according to E. Beck, G. Ludwig, E.A. Auserwald, B. Reiss and H. Schalle "Nucleotide sequence and exact location of the neomycin phosphotransferase gene from transposon Tn5," Gene 19:327-336 (1982); available from P.L. Biochemicals) containing the coding sequences for the neomycin phosphotransferase II gene, was excised, ligated to Eco RI linkers (8mer) and cloned into the EcoRI site of pUC8.

6. pEV1neo(+): The 1kb EcoRI fragment from pRRNEO was cloned into the EcoRI site of pEVI. The orientation of the plasmid is am 5'-NPTII(5-3)-am 3'. This plasmid contains an out-of-frame ATG at 16 bases 5' to the major translation start of the NPTII gene.

7. pAFI: pUC8 with a filled in EcoRI site.

8. pEV2neo: The BamHI fragment of pEVIneo was cloned into the BamHI site of plasmid pAFI.

9. pEV2AGX: A 9kb XhoI fragment (containing the complete glucoamylase gene) obtained from lambda AG111 (see above) was cloned into the SalI site of pEV2neo.

10. pNEObal-3: pNEO was opened with BglII and resected with Bal31 nuclease. EcoRI linkers - (8mer) were added and the plasmid was closed. The specific plasmid contains an EcoRI linker attached immediately adjacent to nucleotide 1543 (numbered according to Beck, et al., supra) in the 5' region of the NPTII gene. This removes the out-of-frame ATG.

6

11. p42-6-1: pNEObal-3 was opened with Aval, filled in with the Klenow fragment of DNA polymerase, and EcoRI linkers (8mer) were added at this site. The plasmid was cut with Eco RI and the resulting ∿1 kb EcoRI fragment (which contains the coding sequence for NPTII) was cloned into the EcoRI site of pUC8.

12. pEV2neobal: The EcoRI fragment of p42-6-1 was introduced to replace the EcoRI fragment of pEV2NEO. The orientation is am 5'-NPTII (5'-3')-am 3'.

13. pEV3neobal: The BamHI fragment of pEV2neobal was introduced into the Bam HI site of pUC18.

14. pEV3neobal ΔXho: The XhoI site in the 3' am sequences of pEV3neobal was filled.

15. pGAN: A 10 kb HindIII-Xba I fragment from AG111 (containing the glucoamylase gene) was cloned between the HindIII and XbaI sites of pEV3neobal ΔXho.)

16. p 42-2-1: A 3.4kb EcoRI fragment (containing the complete glucoamylase gene) obtained from lambda AG111 was cloned into the EcoRI site of pUC8.

17. p50-1-16: p42-2-1 was cut with BssHII and resected with Ba131 nuclease. XhoI linkers - (CCTCGAGG) were added and the plasmid was closed. The specific plasmid contains an XhoI linker attached immediately adjacent to nucleotide -37 (numbered relative to the normal translation start of the glucoamylase gene as +1).

18. pUC8ΔSal: The SalI site of pUC8 was filled.

19. p56-4-7: The EcoRI fragment of p50-1-16 (containing the Bal31 delted glucoamylase gene) was moved to the Eco RI site of pUC8ΔSal.

20. pEVG: p56-4-7 was cut with SalI, filled in, XhoI linkers (8mer) added, cut with XhoI, the large piece isolated and closed.

21. pEVGneobal: pEV2neobal was cut with EcoRI, filled in, XhoI linkers (8mer) added, cut with Xho I, and the EcoRI fragment (containing the NPTII coding region now bounded by XhoI sites) was gel purified and inserted (in the proper orientation) into the XhoI site of pEVG.


## C. TRANSFORMATION

A. niger (or A. awamori) were grown on agar slants (consisting of 2% glucose, 1% peptone, 2% agar) for 8-12 days at 30°C. Conidia were suspended in a solution of 0.005% Nonidet P-40. The conidial suspension was shaken in a tube with glass beads to break up the conidial chains, and then used to inoculate a flask of complete medium (solution 9) (100 ml in a 500 ml Erlenmeyer flask) to a concentration of 5-10 × $10^5$ conidia/ml for incubation at 30°C, 200 rpm, 24 hours. Thereafter, mycelia were harvested by filtering onto a double layer of cheesecloth and washed with KMP (solution 1), with the excess liquid squeezed out. The mycelia were added to a sterile 500 ml flask with 50 ml KMP, 500 mg Novozym 234 - (Novo Laboratories, Wilton, CT 06897) and incubated at 30°C at 70 rpm overnight. To separate spheroplasts from mycelial cell debris, the solution was filtered through a double layer of cheesecloth and glass wool into conical centrifuge tubes, then the filtrate was centrifuged at 1800 rpm for 15 min in a bench top centrifuge. The supernatant was discarded and the pellet of spheroplasts was washed two times (in order to remove the Novozym) by resuspension in KMP and recentrifugation.

For transformation, 5-50 ug DNA in 20 ul 0.01M Tris, 0.001M EDTA (pH 8.0) was added to 6.2 ul heparin solution, and incubated at room temperature for about 10 minutes. The pellet of spheroplasts from the recentrifugation was resuspended in KMPC to 5 × $10^7$/ml, and 200 ul of the spheroplast suspension was added to the DNA with 50 μl PPC, mixed gently and incubated on ice for 30 minutes. Then, 1 ml PPC was added and the transformation mix added to 50 ml of regeneration agar at 50°C containing 0.5 mg G418/ml. (The G418, as 125 ul of 200 mg G418/ml stock solution was added just before adding the spheroplasts.) This mixture was swirled and poured into 4 petri dishes, cooled quickly, then incubated at 30°C for 4 hours. The plates were then overlayed with overlay agar (containing about 3 mg G418/ml) with the number of mls equal to the weight of the regeneration agar mixture added to the dish. The dishes were incubated at 30°C, and transformants appeared in 3 to 7 days.

P. chrysogenum was transformed by the same procedure as above except that the regeneration agar contained no G418 and the overlay was carried out in two steps: the first overlay was with an equal volume of overlay agar containing 0.6 mg/ml G418 at 4 hours and this overlay was repeated at 20 hours.

## D. DETERMINATION OF PLASMID COPY NUMBER

Plasmid copy number was determined by two procedures. In the first instance, colony screening of the transformants was performed. More usually, DNA isolated from the transformants by miniprep was digested with appropriate restriction enzymes, and subjected to Southern hybridization. Where possible, a probe was employed which contained a segment of (single copy) A. niger DNA (for use as an internal standard), as well as a segment of the input plasmid.

## E. DETERMINATION OF GLUCOAMYLASE

Starting with a conidial inoculum, cultures were grown at 30°C on a shaking bath at 200 rpm (1" gyrorotary revolution) with 50 ml of growth medium per silanized 250 erlenmeyer flask. Culture media consisted of 1 $^x$ mineral salts, 0.5% yeast extract, 0.5% tryptone and 10% soluble starch (Lintner/potato starch, Sigma).

Samples were removed (sterile filtered) and incubated with 4% soluble starch in 20 mM sodium citrate - (pH 5.0) at 60°C for 15-60 minutes (incubation time was chosen so as not to convert more than 10% of substrate). Following deproteinization, the free glucose produced was assayed colorimetrically using the glucose oxidase. One unit of enzyme is that amount necessary to produce 1 gm of glucose per hour.

## F. RESULTS

### I. TRANSFORMATION

As shown in Table I, a number of plasmids were able to transform A. niger ATCC 1015. Both pEV2neo and pEV2neobal gave rise to transformants, and pEV2neobal gave rise to multicopy integration of the plasmid. This result is somewhat surprising as one would have expected that pEV2neo (which contains an out-of-frame ATG 5' to the authentic start) would have functioned less efficiently than pEV2neobal. This plasmid would have been expected to be present at a higher copy number in order to compensate for the - (presumed) poorer level of expression. Transformation frequencies were approximately ten transformants or less per microgram of DNA, but higher frequencies may be obtained. The plasmids pEV2neobal, pEVGneobal, and pGAN were also capable of transforming P. chrysogenum at approximately the same frequency as observed for A. niger, and the first and third plasmids listed have also been transformed into A. awamori.

The plasmid pGAN was deposited at the ATCC on 19 November 1985 and given deposit Number 53331.

### TABLE I

| Plasmid | Amount of DNA (ug) | Transformants per ug | Autonomous | Percentage* Single-Copy | Multicopy |
|---------|-------------------|---------------------|------------|-------------------------|-----------|
| pEV2neo | 5 | 2-10 | 17 | 83 | 0 |
| pEV2neobal | 5-50 | 2-10 | 0 | 40 | 60 |
| pGAN | 5-50 | 0.1 | 0 | 30 | 70 |
| pEV2AGX | 5-50 | <0.1 | 0 | 100 | 0 |

* The percentage of multicopy integrants may be over-estimated, as not all transformants were examined (rapidly growing transformants were found to be more likely to contain multicopy plasmid integration and it was primarily these colonies that were examined).

Four lines of evidence demonstrate that the plasmids have been integrated as tandem repeats: 1) In Southern hybridization, no band corresponding to the autonomous form of the plasmid was observed if unrestricted DNA isolated from the transformant was probed; 2) Upon digestion of the DNA with an enzyme which cut the plasmid a single time, a band of DNA was observed which corresponds in size to the intact

8

plasmid; 3) while the plasmid was difficult to recover into E. coli if uncut DNA from the A. niger. transformant was employed, the plasmid was recovered if the DNA was first cut with the appropriate restriction enzyme and religated prior to transformation; and 4) the junction fragments were isolated where the plasmid had been integrated by cutting the DNA of the transformant with the appropriate restriction enzymes and religating prior to transformation of E. coli.

When the A. niger glucoamylase gene was introduced into pEV2neo and pEV2neobal (to produce pEV2AGX and pGAN, respectively); pGAN (the vector containing pEV2neobal) but not pEV2AGX (the vector containing pEV2neo), evidenced multicopy integration. These results indicate that pEV2neobal was capable of directing multicopy integration, even when the vector contained other sequences.

The relative proportion of multicopy integration versus single copy integration (or autonomous replication) varied somewhat among experiments, however, generally about 50% of the transformants of pEV2neobal or pGAN examined were multicopy.

The multicopy transformants, once isolated, were extremely stable. The plasmids were maintained without the need for any selective pressure. No gross differences were observed in the growth of the transformants or the wild type untransformed cells.

As demonstrated by Paietta and Marzluf (Mol. Cell. Biol. 5:1554-1559 (1985), pBR322 sequences are not required in order to transform filamentous fungi. Thus, the plasmid pEV2neobal may be cleaved with the restriction enzyme BamH1, which separates the expression cassette from all pBR322-derived sequences. The purified expression cassette is suitable for transformation of A. niger to G418-resistance, and to provide transformants which contain no pBR322-derived sequences.


## II. ENZYME OVERPRODUCTION

Numerous transformants of A. niger containing multicopy tandem integrants of pGAN were assayed for the production of glucoamylase. As may be observed in Table II, some correlation exists between the copy number of the plasmids and the increased level of expression of glucoamylase. One strain, A03 - (transformed with pGAN), produced about 5 times more glucoamylase than either a control strain, AG10 - (transformed with pEV2neobal), or the untransformed parent.

## TABLE II

| Transformant | Plasmid Copy Number | Glucoamylase (% of Control) |
|---|---|---|
| Experiment I* | | |
| Wild-Type | – | 100 |
| AO12 (pGAN) | 1 | 112 |
| AP4 " | 3 | 180 |
| AP3 " | 3 | 138 |
| AO1 " | 3 | 451 |
| AO3 " | 5 | 600 |
| AO5 " | 5 | 364 |
| AP2 " | 5 | 348 |
| AO14 " | 10 | 348 |
| Experiment II** | | |
| Wild-type | – | 100 |
| A6-1 (pEV2AGX) | 1 | 146 |
| A6-2 " | 1 | 47.2 |
| A3-3 " | 1 | 86.6 |
| A6-7 " | 1 | 173 |

* Cultures were grown for seven days on 10% starch before assay. 100% = 0.47 Units/ml, with one Unit = 1 gram glucose per hour under conditions essentially as described in U.S. Patent 3,301,768 (4% starch, 80 mM sodium acetate pH 4.3, 60°C; instead of 20 mM sodium citrate, pH 5.0)

** Cultures were grown for 3 days on 2% starch before assay. 100% = 0.0127 Units/ml (note: the reduced level of expression of the wild type in this experiment is due to the lower amount of starch employed, as well as the shorter cultivation period).

In accordance with the present invention, novel hybrid DNA constructs are provided which can be stably maintained in filamentous fungi, either as an extrachromosomal element or integrated into the host genome. The constructs are relatively easy to produce, often utilizing commonly available DNA segments for one or more of the DNA segments. They are also stable after transformation, and exhibit a broad host range within filamentous fungi. These constructs are capable of accepting various DNA sequences, such as for structural genes, permitting the introduction of such structural genes into fungi. The quantities of proteins encoded by structural genes can be controlled, and if desired, increased to substantially higher levels than typically found in fungi.

While the above provides a full and complete disclosure of the preferred embodiments of the invention, various modifications, alternate constructions, and equivalents may be employed without departing from the true spirit and scope of the invention. Therefore, the above description should not construed as limiting the scope of the invention.

**Claims**

1. A DNA construct comprising an expression cassette controlling the expression of a structural gene encoding a polypeptide capable of functioning as a selectable marker in a filamentous fungus, said expression cassette comprising a fungal promoter sequence not natively associated with said structural gene, wherein said hybrid DNA composition is capable of stable maintenance in said filamentous fungus.

2. A DNA construct according to claim 1, wherein the entire sequence of said construct is not native to the species of said fungus.

3. A DNA construct according to claim 1, wherein said gene encodes for neomycin phosphotransferase II.

4. A filamentous fungal host containing a hybrid DNA construct comprising a gene encoding a selectable marker, said gene placed in the correct orientation for transcription under control of a fungal promoter sequence and said gene followed by a fungal terminator sequence, wherein said gene is not natively associated with at least one of the sequences and said construct is stably maintained in said host as an extrachromosomal element or by integration into the genome of said host.

5. A method for stably transforming a filamentous fungus comprising the steps of:

forming a vector by inserting a structural gene encoding a selectable marker functional in said fungus into an expression cassette comprising a fungal promoter sequence and terminator sequence, both sequences capable of controlling the expression of the inserted structural gene, wherein said structural gene is not natively associated with at least one of said promoter or terminator sequences;

transforming said fungus with said vector to form transformants;

growing said transformants under conditions requiring, for survival, functional expression of said structural gene; and

isolating surviving transformants.

6. A method of modifying the level of a polypeptide produced by a filamentous fungus, said method comprising the steps of:

i) forming a vector comprising a first expression cassette capable of controlling expression of a selection gene encoding a selectable marker, said expression cassette comprises a fungal promoter sequence, wherein at least one of said sequences is not natively associated with said first structural gene;

ii) joining to said first expression cassette a second expression cassette capable of controlling expression of a structural gene encoding said polypeptide;

iii) transforming said fungus with the vector to form transformants; and

iv) growing said transformants under conditions suitable for expression of said second structural gene.

7. A method of claim 6, wherein said polypeptide is glucoamylase, which is naturally produced by a wild-type strain of said fungus.

8. A hybrid DNA vector including a first expression cassette comprising a neomycin phosphotransferase II gene under transcriptional initiation and termination control of fungal promotion and termination sequences, wherein said vector is capable of stable transformation into a filamentous fungus.

9. A hybrid DNA vector according to claim 8, wherein said fungal promoter and terminator sequences are from a glucoamylase gene or a glutamate dehydrogenase gene.